# EUROPEAN PATENT APPLICATION

(11) **EP 2 602 279 A1**
(43) Date of publication of application: **12.06.2013**
(21) Application number: 12008103.9
(22) Date of filing: 04.12.2012
(51) Int. Cl.: C08G 77/38, C08L 83/06

(54) **Silicone-modified wax, composition and cosmetic preparation containing the same, and production method of silicone-modified wax**

(30) Priority: 07.12.2011 JP 2011268374
(71) Applicant: Shin-Etsu Chemical Co., Ltd., Chiyoda-ku, Tokyo (JP)
(72) Inventor: Iyoku, Hiromi, Annaka-shi, Gunma-ken (JP)
(74) Representative: Wibbelmann, Jobst

(57) **Abstract**

The present invention provides a silicone-modified wax obtained by subjecting an esterification reaction product obtained from a polyhydric alcohol having one or two alkenyl groups in one molecule and a higher fatty acid, and an organohydrogenpolysiloxane containing at least one methyl group, to a hydrosilylation reaction; wherein the silicone-modified wax is solid or in a grease state exhibiting a thixotropy, at 25°C. There can be a silicone-modified wax, a production method thereof, and a composition and a cosmetic preparation each containing the silicone-modified wax, which silicone-modified wax is solid or in a grease state exhibiting a thixotropy, at 25°C, and is high in compatibility with oil-based agents such as volatile and nonvolatile silicone oils, hydrocarbon oils, ester oils, natural animal oils and plant oils, and the like.

## Description

### BACKGROUND OF THE INVENTION

### 1. Field of the Invention

The present invention relates to a silicone-modified wax, a composition and a cosmetic preparation each containing the same, and a production method of the silicone-modified wax.

### 2. Description of the Related Art

Oil-based thickeners such as a wax or a gelling agent (hereinafter collectively called a "thickener") are each used for the purpose of increasing a viscosity of an oil-based component in a cosmetic preparation to improve a usability, stability and appearance of the cosmetic preparation. Widely used as the oil-based thickeners are: hydrocarbon-based waxes such as ceresin, polyethylene and ozokerite; waxes such as carnauba wax and candelilla wax; and polysaccharide fatty acid esters such as sucrose palmitic acid ester.

When these thickeners are each to be blended in a cosmetic preparation, the thickeners are each obliged to be once melted by typically heating it up to a temperature equal to or higher than its melting point. As such, higher melting points of thickeners to be blended occasionally lead to degradation or decomposition of other components in cosmetic preparations. Thus, the thickeners are desired to have lower melting points, particularly at or lower than 100°C, respectively. Unfortunately, thickeners having lower melting points are typically low in thickening effect, and occasionally cause viscosity reduction or phase separation of a cosmetic preparation in summer, for example.

Further, it is desired for a thickener to be excellent in compatibility with a low-viscosity oil-based agent. Inferior compatibilities occasionally lead to timewise separation between a thickener and an oil-based agent of each cosmetic preparation, and to a grown crystal of the thickener upon cooling to deteriorate an external appearance, a feeling, and the like of the cosmetic preparation. Relatedly, silicone oils have been frequently used as oil-based agents for cosmetic preparations, because of excellent properties of each silicone oil, such as a non-viscous feeling, an excellent spread, an excellent water repellency, and the like. Among them, volatile silicone oils have been frequently used to each constitute a cosmetic preparation which is free of stickiness and has a light feeling. Unfortunately, silicone oils are inferior in compatibility with typical oil-based agents and in affinity with thickeners, thereby tending to cause the above problems.

To solve the above problems, thickeners have been developed which are capable of thickening volatile silicone oils, respectively (Patent Document 1 and Patent Document 2). However, it is difficult for them to smoothly thicken such a silicone oil by the applicable thickener by itself, and the thickened product to be obtained then has lacked a smoothness to be demanded for a cosmetic preparation. This has resulted in a proposal of a silicone-modified olefin-based wax intended to solve the problems (Patent Document 3).

When the above thickener is to be blended into a cosmetic preparation, the former is heated and dissolved together with an oil-based agent(s). Although the temperature for heating and dissolving is unexplained, such a temperature is typically at or above a melting point of the wax. Further, it is described that, the silicone-modified olefin-based wax is heated and dissolved together with the oil-based agent(s), and they are subsequently cooled down to a room temperature, thereby obtaining a silicone-based composition, which composition is solidified at a room temperature and exhibits no fluidities, and which composition is usable as a cosmetic preparation (Patent Document 3, paragraph [0195]). However, it appears to be possible for this composition to be improved in spreading property, and a feeling.

Patent Document 1: JP4664991B2
Patent Document 2: WO2004/110393A
Patent Document 3: JP2008-174571A

### SUMMARY OF THE INVENTION

The present invention has been carried out in view of the above circumstances, and it is therefore an object of the present invention to provide a silicone-modified wax, a production method thereof, and a composition and a cosmetic preparation each containing the silicone-modified wax, which silicone-modified wax is high in compatibility with oil-based agents such as volatile and nonvolatile silicone oils, hydrocarbon oils, ester oils, natural animal oils and plant oils, and the like, in a manner to be capable of thickening various oil-based agents to turn them into a pasty or solid composition which is smooth and excellent in spread.

To solve the above problem, the present invention provides a silicone-modified wax obtained by subjecting an esterification reaction product obtained from a polyhydric alcohol having one or two alkenyl groups in one molecule and a higher fatty acid, and an organohydrogenpolysiloxane containing at least one methyl group, to a hydrosilylation reaction;
wherein the silicone-modified wax is solid or in a grease state exhibiting a thixotropy, at 25°C.

Such a silicone-modified wax of the present invention is particularly useful as a thickener to be blended in a cosmetic preparation, in that the silicone-modified wax is high in compatibility with oil-based agents such as volatile and nonvolatile silicone oils, hydrocarbon oils, ester oils, natural animal oils and plant oils, and the like, in a manner to be capable of making various oil-based agents to be pasty or solidifying them. Further, compositions each containing the silicone-modified wax of the present invention are made to be pasty or solid compositions which are smooth and excellent in spread.

It is desirable that the polyhydric alcohol having one or two alkenyl groups in one molecule is a compound having a linear or branched polyglycerin structure;
that the higher fatty acid is a saturated linear higher fatty acid having 12 to 30 carbon atoms; and
that the organohydrogenpolysiloxane is an organohydrogenpolysiloxane represented by the following general formula (A), 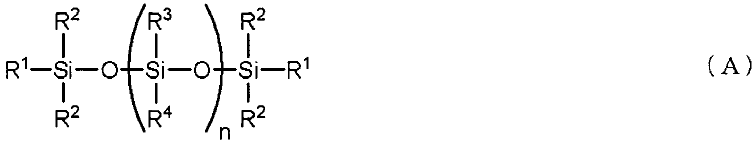 wherein R¹, R², R³, and R⁴ each independently represent:
a hydrogen atom; or
an identical or different linear or branched alkyl group or alkoxy group having 1 to 22 carbon atoms, or an identical or different aryl group having 6 to 22 carbon atoms, or an identical or different fluorine-substituted alkyl group, such that each group may have a coupled group or substitutional group having a branched polyglycerol chain;
wherein at least one of R¹ and R³ represents a hydrogen atom, at least one of R² and R⁴ represents a methyl group, and n represents an integer of 0 to 100.

Such a silicone-modified wax is higher in compatibility with oil-based agents such as volatile and nonvolatile silicone oils, hydrocarbon oils, ester oils, natural animal oils and plant oils, and the like, in a manner to be capable of assuredly making various oil-based agents to be pasty or solidifying them.

Further, it is possible that the silicone-modified wax has a melting point (at an ordinary pressure) between 40°C and 100°C.

Since the silicone-modified wax of the present invention is thus made to have a melting point between 40°C and 100°C, the silicone-modified wax is preferable in that the same is free of possibility to decrease a viscosity of a cosmetic preparation or to cause a phase separation thereof such as in summer, and is free of possibility to cause degradation or decomposition of other components in the cosmetic preparation.

Furthermore, the present invention provides a composition containing the above silicone-modified wax in an amount between 5 and 40 mass%, wherein the composition is in a paste state or solid state at 25°C and at an ordinary pressure.

Such a silicone-modified wax of the present invention is high in compatibility with oil-based agents such as silicone oils and the like, and is high in thickening effect, in a manner to be capable of turning the oil-based agents into pasty or solid compositions (at 25°C at an ordinary pressure) which are smooth and excellent in spread, such that the compositions can each be preferably blended in a cosmetic preparation.

Moreover, the present invention provides a cosmetic preparation containing the above silicone-modified wax.

The silicone-modified wax of the present invention is high in compatibility with various oil-based agents to be used for cosmetic preparations in a manner to be capable of turning the oil-based agents into a pasty or solid composition which is smooth and excellent in spread, so that the composition can be preferably used for a cosmetic preparation. Further, the silicone-modified wax of the present invention is capable of achieving a melting point between 40 and 100°C, preferably between 50 and 80°C, so that the same is free of possibility to decrease a viscosity of a cosmetic preparation or to cause a phase separation thereof such as in summer, and is free of possibility to cause degradation or decomposition of other components in the cosmetic preparation.

Furthermore, the present invention provides a production method of a silicone-modified wax, comprising:
(I) a step of subjecting a polyhydric alcohol having one or two alkenyl groups in one molecule and a higher fatty acid, to an esterification reaction in the presence of a catalyst, thereby obtaining an esterification reaction product; and
(II) a step of subjecting the esterification reaction product and an organohydrogenpolysiloxane containing at least one methyl group, to a hydrosilylation reaction in the presence of a catalyst.

According to such a production method of a silicone-modified wax of the present invention, it is enabled to produce such a silicone-modified wax which is high in compatibility with oil-based agents such as volatile and nonvolatile silicone oils, hydrocarbon oils, ester oils, natural animal oils and plant oils, and the like, in a manner to be capable of making various oil-based agents to be pasty or solidifying them.

It is preferable that the production method further comprises the steps of:
adopting, as the polyhydric alcohol having one or two alkenyl groups in one molecule, a compound having a linear or branched polyglycerin structure,;
adopting, as the higher fatty acid, a saturated linear higher fatty acid having 12 to 30 carbon atoms; and
adopting, as the organohydrogenpolysiloxane, an organohydrogenpolysiloxane represented by the following general formula (A), 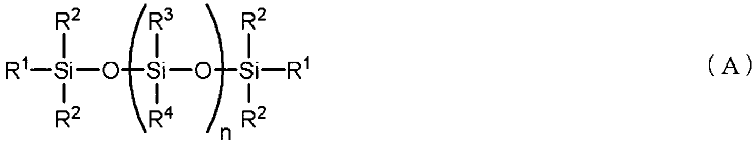
wherein R¹, R², R³, and R⁴ each independently represent:
   a hydrogen atom; or
   an identical or different linear or branched alkyl group or alkoxy group having 1 to 22 carbon atoms, or an identical or different aryl group having 6 to 22 carbon atoms, or an identical or different fluorine-substituted alkyl group, such that each group may have a coupled group or substitutional group having a branched polyglycerol chain;
   wherein at least one of R¹ and R³ represents a hydrogen atom, at least one of R² and R⁴ represents a methyl group, and n represents an integer of 0 to 100.

Such a production method of a silicone-modified wax is capable of producing a silicone-modified wax which is higher in compatibility with oil-based agents such as volatile and nonvolatile silicone oils, hydrocarbon oils, ester oils, natural animal oils and plant oils, and the like, in a manner to be capable of assuredly making various oil-based agents to be pasty or solidifying them.

As described above, the silicone-modified wax of the present invention is high in compatibility with oil-based agents such as volatile and nonvolatile silicone oils, hydrocarbon oils, ester oils, natural animal oils and plant oils, and the like, in a manner to be capable of turning various oil-based agents into a pasty or solid composition which is smooth and excellent in spread. Further, the silicone-modified wax is made to have a melting point between 40°C and 100°C, such that the silicone-modified wax is capable of preventing a viscosity reduction or a phase separation of a cosmetic preparation such as in summer and capable of preventing degradation, decomposition, and the like of other components upon preparing the silicone-modified wax (i.e., upon heating and melting it) into a composition, thereby allowing the composition to be preferably used as a cosmetic preparation.

### DETAILED DESCRIPTION OF THE PREFERRED EMBODIMENTS

The present invention will be explained hereinafter in detail.
As described above, there has been sought for a thickener which is high in compatibility with volatile and nonvolatile silicone oils, hydrocarbon oils, ester oils, natural animal oils and plant oils, and the like, in a manner to be capable of making such various oil-based agents to be pasty or solidifying them.

The present inventors have earnestly conducted investigations so as to solve the above problem, and have resultingly found out that such a silicone-modified wax obtained by subjecting an esterification reaction product obtained from a polyhydric alcohol having one or two alkenyl groups in one molecule and a higher fatty acid, and an organohydrogenpolysiloxane containing at least one methyl group, to a hydrosilylation reaction, wherein the silicone-modified wax is solid or in a grease state exhibiting a thixotropy, at 25°C, is high in compatibility with volatile and nonvolatile silicone oils, hydrocarbon oils, ester oils, natural animal oils and plant oils, and the like, in a manner to be capable of thickening such various oil-based agents to turn them into a pasty or solid composition which is smooth and excellent in spread.

The present invention will be described hereinafter in more detail.
Namely, the present invention provides a silicone-modified wax obtained by subjecting an esterification reaction product obtained from a polyhydric alcohol having one or two alkenyl groups in one molecule and a higher fatty acid, and an organohydrogenpolysiloxane containing at least one methyl group, to a hydrosilylation reaction; wherein the silicone-modified wax is solid or in a grease state exhibiting a thixotropy, at 25°C.

Further, the present invention provides a production method of a silicone-modified wax, comprising:
(I) a step of subjecting a polyhydric alcohol having one or two alkenyl groups in one molecule and a higher fatty acid, to an esterification reaction in the presence of a catalyst, thereby obtaining an esterification reaction product; and
(II) a step of subjecting the esterification reaction product and an organohydrogenpolysiloxane containing at least one methyl group, to a hydrosilylation reaction in the presence of a catalyst.

Examples of the polyhydric alcohol having one or two alkenyl groups in one molecule to be used in the present invention, include linear or branched polyglycerins each having any one of the structural formulae (B), (C), (D), and (E), and each containing one or two alkenyl groups:

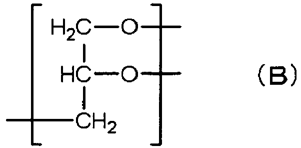

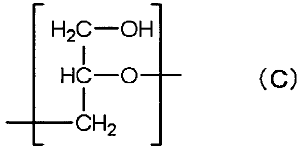

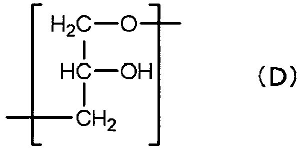

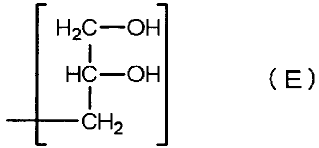

Further, it is possible to preferably use such a polyhydric alcohol having an alkenyl group(s) at its end(s), in a manner to preferably use a triglycerin derivative or a tetraglycerin derivative, for example, such as follows, 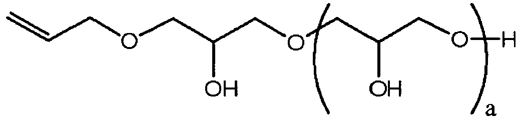 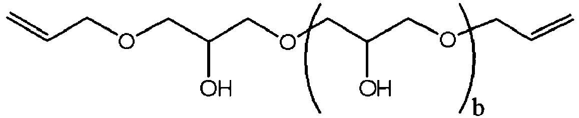 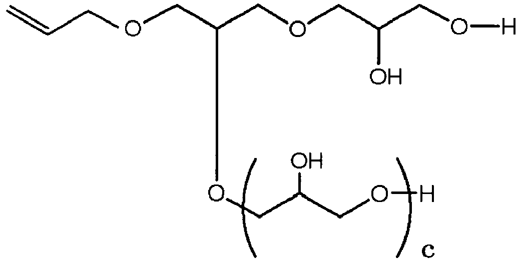 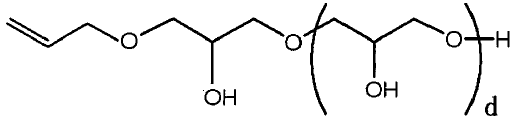 wherein a, b, c, and d represent each 2 or 3.

Further, the polyhydric alcohol having one or two alkenyl groups to be used in the present invention may have a linear or branched ether structure, or even a cyclic ether structure.

As a production method of these polyhydric alcohols each containing an alkenyl group(s), it is possible to react glycerin, or a polyglycerin such as diglycerin or triglycerin, with allyl glycidyl ether in the presence of an alkali catalyst such as potassium hydroxide, thereby obtaining an applicable polyhydric alcohol. Preferably, the reaction temperature is controlled to be between 60 and 120°C. After the reaction, the alkali catalyst is neutralized, followed by distillation removal of low-boiling fractions, thereby allowing to obtain an intended product (polyhydric alcohol having one or two alkenyl groups).

The higher fatty acid to be used in the present invention is a linear saturated fatty acid, preferably having 12 to 30 carbon atoms, more preferably having 18 to 26 carbon atoms. Examples of the higher fatty acid include myristic acid, palmitic acid, stearic acid, oleic acid, arachidic acid, behenic acid, lignoceric acid, cerotic acid, montanoic acid, and melissic acid, which are each used solely in one kind, or mixedly in two or more kinds.

Then, the polyhydric alcohol having one or two alkenyl groups in one molecule and the higher fatty acid are charged into a reaction vessel, in a manner to conduct an esterification reaction at a temperature between 160 and 240°C for about 5 to 30 hours in an inert gas flow while eliminating water to be produced by the reaction, followed by purifying treatments in the usual manner such as decoloration, deodorization, and the like after completion of the esterification reaction, thereby enabling to obtain an esterification reaction product.
Upon conducting the esterification reaction, it is possible to use an acid catalyst, a metal catalyst, and a refluxing solvent, as required.

Examples of the organohydrogenpolysiloxane containing at least one methyl group to be subjected to the hydrosilylation reaction with the esterification reaction product, include such an organopolysiloxane having at least one hydrogen atom and at least one methyl group each directly bonded to an applicable silicon atom in one molecule, which organohydrogenpolysiloxane is represented by the following structural formula (A), 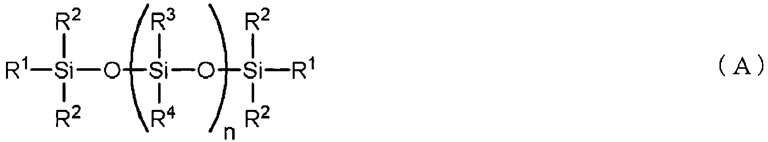 wherein R¹, R², R³, and R⁴ each independently represent:
a hydrogen atom; or
an identical or different linear or branched alkyl group or alkoxy group having 1 to 22 carbon atoms, or an identical or different aryl group having 6 to 22 carbon atoms, or an identical or different fluorine-substituted alkyl group, such that each group may have a coupled group or substitutional group having a branched polyglycerol chain;
wherein at least one of R¹ and R³ represents a hydrogen atom, at least one of R² and R⁴ represents a methyl group, and n represents an integer of 0 to 100.
Preferable as the R¹ represents a hydrogen atom, or an alkyl group having 1 to 6 carbon atoms, and particularly preferably a hydrogen atom, a methyl group, or a butyl group. Preferable as the R² represents an alkyl group having 1 to 6 carbon atoms, and particularly a methyl group. Preferable as the R³ represents a hydrogen atom, or an alkyl group having 1 to 6 carbon atoms, and particularly preferably a hydrogen atom, or a methyl group. Preferable as the R⁴ is an alkyl group having 1 to 6 carbon atoms, and particularly a methyl group.

Preferable among them is such an organohydrogenpolysiloxane having 1 to 5 hydrogen atoms, more preferably 1 to 2 hydrogen atoms, and particularly preferably one hydrogen atom, bonded to a silicon atom(s) (Si-H group(s)). More preferable is an α-hydrogen organopolysiloxane having a Si-H group(s) at an end(s) of the molecule moiety.
In the above, n represents an integer of 0 to 100, and preferably an integer of 0 to 50, more preferably 1 to 20, and particularly preferably 1 to 10.

Specifically, examples of the organohydrogenpolysiloxane include those represented by the following formulae, respectively: 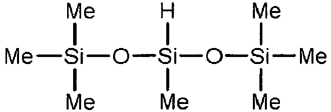 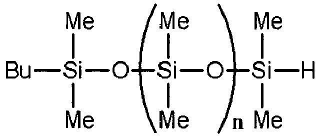 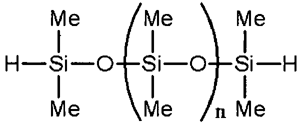 wherein n represents described above.

As a composition production method of the organohydrogenpolysiloxane to be used in the present invention, it is possible to charge hexamethylsiloxane, octamethylcyclotetrasiloxane (D4), and tetramethyltetrahydrogencyclotetrasiloxane (H4) into a reaction vessel, to add an acid catalyst thereinto, and to conduct stirring at an ordinary temperature overnight, followed by conduction of purifying treatments such as neutralization, decoloration, and deodorization, thereby obtaining methylhydrogensiloxane.

The methylhydrogen organopolysiloxane is added to the glycerin derivative having an alkenyl group (i.e., the esterification reaction product), at a molar ratio of Si-H group/alkenyl group, between 0.5 inclusive and 1.5 exclusive, preferably between 0.8 inclusive and 1.2 exclusive.

Such an addition reaction is to be preferably conducted in the presence of a platinum catalyst or rhodium catalyst. Examples of preferable catalysts include chloroplatinic acid, alcohol-modified chloroplatinic acid, a chloroplatinic acid-vinylsiloxane complex, and the like.
The addition reaction may be conducted in an organic solvent, as required. Examples of such an organic solvent include aliphatic alcohols such as methanol, ethanol, 2-propanol, butanol, and the like, aromatic hydrocarbons such as toluene, xylene, and the like, aliphatic or alicyclic hydrocarbons such as n-pentane, n-hexane, cyclohexane, and the like, hydrocarbon halogenides such as dichloromethane, chloroform, and carbon tetrachloride, and the like.

Although the condition for the addition reaction is not particularly limited, it is preferable to conduct the reaction for 1 to 10 hours under reflux.
After the addition reaction, it is possible to conduct elimination of allylether group by weak hydrochloric acid, or to conduct alkylation by a hydrogenation reaction, each by a known technique. Further, it is possible to add, as an antioxidant, tocopherol or BHT (dibutylhydroxytoluene).

The silicone-modified wax of the present invention is solid or in a grease state exhibiting a thixotropy, at 25°C, and the silicone-modified wax is capable of achieving a melting point between 40 and 100°C, preferably between 50 and 80°C. Melting points at 40°C or higher of the waxes typically lead to sufficient thickening effects, in a manner to be free of possibility to decrease a viscosity of a cosmetic preparation or to cause a phase separation thereof such as in summer. In turn, melting points at 100°C or lower are preferably free of possibility to cause degradation or decomposition of other components in the cosmetic preparation.

Further, the silicone-modified wax of the present invention is to preferably have a weight-average molecular weight between 1,000 and 20,000 as measured by GPC.

### [Composition]

The composition according to the present invention contains the silicone-modified wax of the present invention in an amount between 5 and 40 mass%. Since the silicone-modified wax thickens various oil-based agents and the like, the former is mixed with various oil-based agents to turn them into a pasty or solid composition which is smooth and excellent in spread at 25°C and an ordinary pressure, by virtue of the higher compatibility of the wax with the various oil-based agents. The composition is a mixture of the silicone-modified wax and a silicone oil to be described later, for example, and can be used in a car wax, various mold lubricants, and the like, in addition to in a cosmetic preparation to be described later.

### [Cosmetic preparation]

The silicone-modified wax (higher fatty acid ester-modified organopolysiloxane wax) of the present invention is usable for various cosmetic preparations, and is particularly suitable as a raw material for cosmetic preparations to be externally applied to a skin or hair such as skin care products, makeup products, hair care products, antiperspirant products, ultraviolet ray protective products. The blending amount of the silicone-modified wax in the cosmetic preparation can be appropriately adjusted within a range of 0.5 to 95 mass%, preferably within a range of 1 to 70 mass% of a total mass, depending on a kind of the cosmetic preparation and a type of a silicone oil to be blended therein. For example, in a cream form cosmetic preparation, the content may range from 0.5 to 10 mass%, preferably from 0.5 to 5 mass% of a total weight of the cosmetic preparation; in a solid or stick-shaped cosmetic preparation containing powdery components in an amount of 25% or less, the content may range from 5 to 70 mass%, preferably from 2 to 50 mass% of a total weight of the cosmetic preparation; and in a solid cosmetic preparation containing powdery components in an amount of 80% or more, the content may range form 1 to 10 mass%, preferably from 1 to 5 mass% of a total weight of the cosmetic preparation.

The silicone-modified wax is to be preferably blended in a cosmetic preparation together with a silicone oil. In the present specification, examples of the silicone oil include not only those silicone compounds which are liquid at an ordinary temperature (25°C), but also those each obtained by dissolving or dispersing a silicone resin, which is resinous or gummy at the ordinary temperature, in an oil-based agent having a low viscosity, such as a conventional silicone oil, hydrocarbon oil, or ester oil. These silicon oils are mixed with the above silicone-modified waxes, thereby each providing a pasty or solid composition which is smooth and excellent in spread.
Preferred examples of the silicone oil include the following,

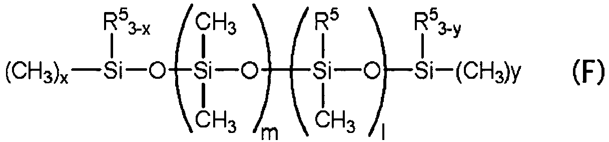

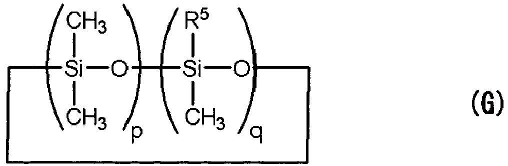

(CH₃)₄-ᵣSi{OSi(CH₃)₃}ᵣ (H)

wherein
R⁵ represents a group selected from a group a consisting of a hydrogen atom, a hydroxyl group, unsubstituted or fluorine-substituted monovalent alkyl groups having 1 to 20 carbon atoms, aryl groups, amino-substituted alkyl groups, alkoxy groups, and a group represented by a general formula (CH₃)₃SiO{(CH₃)₂SiO}ₛSi (CH₃)₂CH₂CH₂-; and
m represents an integer of from 0 to 1,000, 1 is an integer of from 0 to 1,000, 1+m is an integer from 1 to 1,000, x, y represent each 0, 1, 2, or 3, p and q represent each an integer of 0 to 8, 3≤p+q≤8, r represents an integer of 1 to 4, and s is an integer of 0 to 500.

Examples of R⁵ include a methyl group, ethyl group, propyl group, butyl group, hexyl group, octyl group, decyl group, dodecyl group, tetradecyl group, hexadecyl group, octadecyl group, trifluoropropyl group, nonafluorohexyl group, heptadecylfluorodecyl group, phenyl group, aminopropyl group, dimethylaminopropyl group, aminoethylaminopropyl group, stearoxy group, butoxy group, ethoxy group, propoxy group, cetyloxy group, myristyloxy group, styryl group, and α-methylstyryl group, where the hexyl group, octyl group, decyl group, dodecyl group, tetradecyl group, hexadecyl group, octadecyl group, trifluoropropyl group, phenyl group, aminopropyl group, and aminoethylaminopropyl group are preferably included.

Examples of the silicone oil include:
organopolysiloxanes which are liquid at room temperature having a low viscosity to a high viscosity, preferably from 0.65 to 1,000,000 mm²/s, such as dimethylpolysiloxane, methylphenylpolysiloxane, methylhydrogenpolysiloxane, dimethylsiloxane/methyphenylsiloxane copolymer; cyclic siloxanes such as octamethylcyclotetrasiloxane (D4), decamethylcyclopentasiloxane (D5), dodecamethylcyclohexasiloxane (D6), tetramethyltetrahydrogencyclotetrasiloxane (H4), and tetramethyltetraphenylcycloterasiloxane; branched siloxanes such as tristrimethylsiloxysilane (M3T), tetrakistrimethylsiloxysilane (M4Q), and tristrimethylsiloxyphenylsilane; higher alkoxy-modified silicones such as stearoxy silicone; alkyl-modified silicones; amino-modified silicones; and fluorine-modified silicones.

One of the preferable silicone resins to be used in a dissolved or dispersed state in a low viscosity oil-based agent is a crosslinked organopolysiloxane swollen with a silicone oil having a viscosity of from 0.65 to 10.0 mm²/s in an amount equal to or more than the weight of the crosslinked organopolysiloxane itself, which crosslinked organopolysiloxane is obtained by reacting an alkylhydrogenpolysiloxane with a crosslinking agent having at least two reactive vinyl-like unsaturated groups in a molecule. Examples of the alkylhydrogenpolysiloxane include methylhydrogenpolysiloxanes which are each linear or each have a partial branched unit, methylhydrogenpolysiloxanes each grafted with an alkyl chain having 6 to 20 carbon atoms, and methylhydrogenpolysiloxanes each grafted with a polyoxyethylene chain. In one molecule, the number of hydrogen atoms bonded to silicon atoms is required to be two or more in an averaged manner. Examples of the crosslinking agent include compounds having two or more of vinyl-like reactive groups such as methylvinylpolysiloxane, α,ω-alkenyldiene, glycerin triallyl ether, polyoxyalkynylated glycerin triallyl ether, trimethylolpropane triallyl ether, and polyoxyalkynylated trimethylolpropane triallyl ether.

The crosslinked organopolysiloxane preferably has, in a cross-linking molecule, at least one kind selected from the group consisting of a polyoxyalkylene moiety, polyglycerin moiety, alkyl moiety, alkenyl moiety, aryl moiety, and fluoroalkyl moiety. Preferable specific examples of the crosslinked organopolysiloxane include those described in JP2-43263A, JP2-214775A, JP2631772B2, JP9-136813A(KSG30), JP2001-342255A, WO03/20828(KSG210), and WO03/24413(KSG40). Using the crosslinked organopolysiloxane allows to expect provision of effects such as an anti-shiny effect, a mat-feel providing effect, an adherence improving effect, and a color migration preventing effect.

Specific examples of the composition comprising the crosslinked organopolysiloxane and the oil-based agent such as silicone oil, hydrocarbon oil, or ester oil, include KSG-15, 16, 17, 18, 21, 210, 31, 32, 33, 34, 310, 320, 330, 340, 41, 42, 43, 44, 710, 810, 820, 830 and 840 (product names produced by Shin-Etsu Chemical Co., Ltd.).

The secondly preferred silicone resin to be used in a dissolved or dispersed state in an oil-based agent having a low viscosity is a silicone resin which is gummy or solid at room temperature and soluble in decamethylcyclopentasiloxane. The preferable gummy silicone resin is such a linear silicone represented by the formula,
(CH₃)₃SiO{(CH₃)₂SiO}ₜ{(CH₃)R⁶SiO}ᵤSi(CH₃)₃, where R⁶ is selected from the group consisting of alkyl groups having 6 to 20 carbon atoms, amino-substituted alkyl groups having 3 to 15 carbon atoms, fluorinated alkyl groups, alkyl groups containing a quaternary ammonium salt group, t is from 1,001 to 20,000, and u is from 0 to 5,000, t+u is from 1,001 to 25,000.

It is preferable that the solid silicone resin is a silicone network compound which is an MQ resin, MDQ resin, MTQ resin, MDTQ resin, TD resin, TQ resin, or TDQ resin, which comprises any combination of a trialkylsiloxy unit (M unit), a dialkylsiloxy unit (D unit), a monoalkylsiloxy unit (T unit), and a tetra functional unit (Q unit). Particularly preferable is a silicone network compound having, in a molecule, at least one moiety selected from the group consisting of pyrrolidone moiety, long alkyl chain moiety, polyoxyalkylene moiety, and fluoroalkyl moiety, (JP2000-234062A, and JP3218872B2).

The thirdly preferred silicone resin to be used in a dissolved or dispersed state in an oil-based agent having a low viscosity is an acrylic silicone resin which is semisolid or solid at a room temperature and is dissolved in a volatile silicone, a volatile hydrocarbon oil, a non-volatile silicone, or a non-volatile hydrocarbon oil. It is preferable that the acrylic silicone resin contains, in a molecule, at least one moiety selected from the group consisting of pyrrolidone moiety, long alkyl chain moiety, polyoxyalkylene moiety, and fluoroalkyl moiety. Such an acrylic silicone resin may have a structure which is in a grafted shape at one of silicone chain and an acrylic chain, or which is in a block shape comprising a silicone chain and an acrylic chain (JP1-319518A, JP2704730B2, JP2767633B2, JP2767636B2, and JP2000-344829A).

To be preferably used among the above silicone oils are: dimethylpolysiloxanes having a viscosity of from 1 to 30 mm²/s (25°C); decamethylcyclopentasiloxane; the crosslinked dimethylpolysiloxane swollen with a silicone oil having a viscosity of from 0.65 to 10.0 mm²/s in an amount equal to or more than the weight of the crosslinked organopolysiloxane itself; the crosslinked polyether-modified silicone; and the acrylic silicone resin dissolved in a volatile oil. These silicone oils may be used solely or as a mixture.

It is preferable that the silicone-modified wax of the present invention and the silicone oil are mixed with each other at a weight ratio of 1:0.01 to 1:4.5, preferably 1:0.5 to 1:20 in a manner to contain the silicone-modified wax in an amount of 5 to 40 mass%, and the composition as a homogeneous paste-like, gel-like, or solid composition is blended in a cosmetic preparation. Although the blending amount of the silicone oil in the entire cosmetic preparation can be appropriately adjusted depending on the type of the cosmetic preparation, such a blending amount is typically in a range of 1 to 70 mass%, preferably in a range of 3 to 60 mass%. Here, the mass of the silicone oil is an amount including a solvent.

In addition to the silicone oil, it is possible to blend one kind or two or more kinds of oil-based agents into the cosmetic preparation of the present invention depending on its intended usage. Insofar as usable in a typical cosmetic preparation, any solid, semi-solid, and liquid oil-based agents are usable. Examples of such oil-based agents include natural animal or plant oils, semi-synthetic oils, hydrocarbon oils, higher alcohol agents, and ester oils.

Examples of the natural animal or plant oils and semi-synthetic oils include avocado oil, linseed oil, almond oil, insect wax, perilla oil, olive oil, cacao butter, kapok wax, kaya oil, carnauba wax, cod-liver oil, candelilla wax, beef tallow, neat's-foot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, bran wax, germ oil, horse fat, persic oil, palm oil, palm kernel oil, castor oil, hydrogenated castor oil, castor oil fatty acid methylester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton suet, peanut oil, lanoline, liquid lanoline, hydrogenated lanoline, lanoline alcohol, hard lanoline, lanoline acetate, isopropyl lanolate, hexyl laurate, POE lanoline alcohol ether, POE lanoline alcohol acetate, polyethylene glycol lanolate, POE hydrogenated lanoline alcohol ether, and egg yolk oil. In the above, POE implies polyoxyethylene.

Examples of the hydrocarbon oils include ozokerite, squalane, squalene, ceresin, paraffin, paraffin wax, polyethylene wax, polyethylene-polypropylene copolymer, liquid paraffin, pristane, polyisobutylene, microcrystalline wax, and vaseline; and examples of the higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, POE cholesterol ether, monostearyl glycerin ether (batyl alcohol), and monooleyl glyceryl ether (cerakyl alcohol).

Examples of the ester oils include diisobutyl adipate, 2-hexyldecyl adipate, di-2-heptylundecyladipate, N-alkyl glycolmonoisostearate, isocetyl isostearate, trimethylolpropane triisostearate, isotridecyl isononanoate, isononyl isononanoate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl malate; and examples of the glyceride oils include acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

Examples of fluorinated oil-based agents include perfluoropolyether, perfluorodecarine, and perfluorooctane.

These oil-based agents are each to be preferably blended in a cosmetic preparation, in an amount within a range of 5 to 95 mass% of a total weight of the cosmetic preparation. Particularly, in case that the cosmetic preparation is in a solid form or solid stick form, each oil-based agent is preferably blended in the cosmetic preparation in an amount between 10 and 50 mass% of the total weight thereof.

Depending on the intended usage, the cosmetic preparation of the present invention may adopt one kind or two or more kinds of compounds each having an alcoholic hydroxyl group in a molecular structure. Examples of those compounds each having an alcoholic hydroxyl group which may be added in the present invention, include lower alcohols such as ethanol and isopropanol; sugar alcohols such as sorbitol, and maltose; sterols such as cholesterol, sitosterol, phytosterol, and lanosterol; polyhydric alcohols such as butylene glycol, propylene glycol, dibutylene glycol, and pentylene glycol. The compounds are each to be preferably blended in a cosmetic preparation, in an amount within a range of 0.1 to 98 mass% of a total weight of the cosmetic preparation.

Depending on the intended usage, the cosmetic preparation of the present invention may adopt water to be blended therein. The blending amount of water is preferably within a range of 1 to 95 mass% of a total amount of the cosmetic preparation.
Further, depending on the intended usage, the cosmetic preparation of the present invention may adopt one kind or two or more kinds of compounds each containing a powder and/or coloring agent.

Usable as the powder are those to be used in typical cosmetic preparations, irrespectively of the shape (spherical, needle-like, or plate-like), particle size (fume-like, fine particle, or pigment grade), and particle structure (porous, non-porous, or the like). Examples of the powder include an inorganic powder, organic powder, powder of surfactant metal salt, colored pigment, pearl pigment, metallic powder pigment, and natural dye.

Specific examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, cericite, muscovite, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salt of tungstenic acid, hydroxyapatite, vermiculite, higilite, bentonite, montmorillonite, hectolitre, zeolite, ceramics powder, dibasic calcium phosphate, alumina, aluminum hydroxide, boron nitride, and silica.

Specific examples of the organic powder include a polyamide powder, polyester powder, polyethylene powder, polypropylene powder, polystyrene powder, polyurethane powder, benzoguanamine powder, polymethylbenzoguanamine powder, tetrafluoroethylene powder, polymethylmethacrylate powder, cellulose powder, silk powder, nylon powder such as Nylon 12 and Nylon 6, a crosslinked silicone fine powder having a structure of cross-linked dimethylsilicone, polymethylsilsesquioxane fine powder, a composite powder where polymethylsilsesquioxane is bonded on cross-linked silicone, styrene/acrylic acid copolymer, divinylbenzene/styrene copolymer, vinyl resin, urea resin, phenol resin, fluororesin, silicone resin, acrylic resin, melamine resin, epoxy resin, polycarbonate resin, microcrystalline fiber powder, starch powder, and lauroyl lysine.

Specific examples of the surfactant metal salt powder (metal soap) include zinc stearate, aluminum stearate, calcium stearate, magnesium stearate, zinc myristate, magnesium myristate, zinc cetyl phosphate, calcium cetyl phosphate, and zinc/sodium cetyl phosphate.

Specific examples of colored pigments include:
inorganic red pigments such as iron oxide, iron hydroxide, and iron titanate; inorganic brown pigments such as γ-iron oxide; inorganic yellow pigments such as iron oxide yellow and loess; inorganic black pigments such as iron oxide black and carbon black; inorganic violet pigments such as manganese violet and cobalt violet; inorganic green pigments such as chromium hydroxide, chromium oxide, cobalt oxide, and cobalt titanate; inorganic blue pigments such as Prussian blue and ultramarine blue; lakes of tar dyes; lakes of natural dyes; and synthetic resin powder obtained by complexing these powders.

Specific examples of the pearl pigments include titanium oxide-coated mica, bismuth oxychloride, titanium oxide-coated bismuth oxychloride, titanium oxide-coated talc, fish scales, and titanium oxide-coated colored mica; and specific examples of the metallic powder pigment include an aluminum powder, copper powder, and stainless steel powder.

Examples of the tar dyes include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and examples of the natural dyes include those selected from among carminic acid, laccaic acid, carthamin, brazilin, and crocin powders.

These powders may be used in a form of a composite of powders, or in a form treated with a typical oil-based agent, silicone oil, fluorine compound, or surfactant, and may be used solely in one kind, or mixedly in two or more kinds as required. The blending amount of the powder is preferably between 0.1 and 99 mass% of the total amount of the cosmetic preparation. Particularly, the blending amount of the powder in case of a powdery solid cosmetic preparation is preferably between 80 and 99 mass% of the total amount of the cosmetic preparation.

Depending on the intended usage, the cosmetic preparation of the present invention may adopt one kind or two or more kinds of surfactants. Examples of such surfactants include anionic, cationic, nonionic, amphoteric ones, and any surfactants are usable insofar as they are used in typical cosmetic preparations, without particularly limited thereto.

As described hereinafter, examples of the anionic surfactant include: fatty acid soaps, such as sodium stearate and triethanolamine palmitate; alkylether carboxylic acids and salts thereof; condensate salts of amino acids and fatty acids; alkanesulfonates; alkenesulfonates; sulfonates of fatty acid ester; sulfonates of fatty acid amides; sulfonates of formalin condensates; alkyl sulfate salts; secondary higher alcohol sulfate salts; alkyl/allyl ether sulfate salts; sulfate salts of fatty acid esters; sulfate salts of fatty acid alkylolamide; sulfate salts such as Turkey Red oil; alkyl phosphates; ether phosphates; alkylallylether phosphates; amide phosphates; and N-acylamino acid-based surfactants. Examples of the cationic surfactants include: amine salts such as alkylamine salts, polyamines, and amino alcohol fatty acid derivatives; alkyl quaternary ammonium salts; aromatic quaternary ammonium salts; pyridium salts; and imidazolium salts.

Examples of the nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkylethers, polyoxypropylene alkylethers, polyoxyethylene alkylphenylethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanolether, polyoxyethylene phytosterolether, polyoxyethylene cholestanolether, polyoxyethylene cholesterylether, polyoxyalkylene-modified organopolysiloxane (see JP7-91389B2, JP7-330907A), polyglycerin-modified organopolysiloxane (see JP62-34039A, JP2613124B2, JP2844453B2, and JP2002-179798A), polyoxyalkylene- and alkyl-co-modified organopolysiloxane (see JP61-90732A, and JP9-59386A), alkanolamide, sugar ethers, and sugar amides; and examples of the amphoteric surfactant include betaine, aminocarboxylate, imidazoline derivatives, and amideamine type. Further, the blending amount of the surfactant is preferably between 0.1 and 20 mass%, particularly preferably between 0.2 and 10 mass% of the total amount of the cosmetic preparation.

Further, it is possible to add, into the cosmetic preparation of the present invention, components used in typical cosmetic preparations, oil-soluble gelling agents, clay minerals modified with organic compounds, resins, antiperspirants, ultraviolet-ray absorbents, ultraviolet-ray absorbing and scattering agents, moisture retention agents, antiseptics, anti-microbial agents, perfumes, salts, antioxidants, pH regulators, chelating agents, refreshing agents, anti-inflammatory agents, skin beautifying components(such as skin whitener, cell activator, rough dry skin improver, blood circulation promoter, skin astringent and anti-seborrheic agent), vitamins, amino acids, nucleic acids, hormones, clathrate compounds, and hair setting agents.

Examples of oil-soluble gelling agents include those gelling agents selected from among: metallic soaps such as aluminum stearate, magnesium stearate, and zinc myristate; amino acid derivatives such as N-lauroyl-L-glutamic acid, α,γ-di-n-butylamine; dextrin fatty acid esters such as dextrin palmitate, dextrin stearate, and dextrin 2-ethylhexane palmitate; fructo-oligosaccharide fatty acid esters such as fructo-oligosaccharide stearate, and inulin 2-ethyl hexanoic acid stearate; sucrose fatty acid esters such as sucrose palmitate, and sucrose stearate; benzylidene derivatives of sorbitol such as monobenzylidene sorbitol and dibenzylidene sorbitol; clay minerals modified with an organic moiety such as dimethylbenzyldodecylammonium montmorillonite clay, and dimethyldioctadecylammonium montmorillonite clay.

Examples of the antiperspirant include those to be selected from among aluminum chlorohydrate, aluminum chloride, aluminum sesquichlorohydrate, zirconyl hydoxychloride, aluminum zirconium hydroxychloride, and aluminum zirconium glycine complex.

Examples of the ultraviolet-ray absorbents include: ultraviolet-ray absorbents of benzoic acid type, such as p-aminobenzoic acid; ultraviolet-ray absorbents of anthranilic acid type, such as methyl anthranilate; ultraviolet-ray absorbents of salicylic acid type, such as methyl salicylate; ultraviolet-ray absorbents of cinnamic acid type, such as octyl p-methoxycinnamate; ultraviolet-ray absorbents of benzophenone type, such as 2,4-dihydroxybenzophenone; ultraviolet-ray absorbents of urocanic acid type, such as ethyl urocanate; and ultraviolet-ray absorbents of dibenzoylmethane type, such as 4-t-butyl-4'-methoxydibenzoylmethane; and examples of the ultraviolet-ray absorbing and scattering agents include ultraviolet-ray absorbing and scattering powders such as a fine powder of titanium oxide, fine powder of iron-containing titanium oxide, fine powder of zinc oxide, fine powder of cerium oxide, a composite thereof, and the like.

Examples of the moisture retention agents include glycerin, sorbitol, propylene glycol, dipropylene glycol, 1,3-butylene glycol, pentylene glycol, glucose, xylitol, maltitol, polyethylene glycol, hyaluronic acid, chondroitin sulfuric acid, pyrrolidone carboxylate, polyoxyethylene methylglycoside, and polyoxypropylene methylglycoside.

Examples of the antiseptics include alkyl paraoxybenzoates, benzoic acid, sodium benzoate, sorbic acid, potassium sorbate, and phenoxyethanol; and examples of the antibacterial agents include benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide, photosensitizer, and phenoxyethanol.

Examples of the antioxidants include tocopherol, butylhydroxyanisole, dibutylhydroxytoluene, and phytin; examples of the pH regulator include lactic acid, citric acid, glycolic acid, succinic acid, oxalic acid, dl-malic acid, potassium carbonate, sodium hydrogen carbonate, and ammonium hydrogen carbonate; examples of the chelating agent include alanine, sodium edetate, sodium polyphosphate, sodium methaphosphate, and phosphoric acid; examples of the refreshing agents include L-menthol and camphor; and examples of the anti-inflammatory agents include arantoin, glycyrrhizic acid and salts thereof, glycyrrhetic acid, stearyl glycyrrhetate, tranexamic acid, and azulene.

Examples of the skin-beautifying components include: whitening agents such as placenta extract, arbutin, glutathione, and saxifrage extract; cell activators such as royal jelly, photosensitizers, cholesterol derivatives, and calf blood extract; rough and dry skin improvers; blood circulation improvers such as nonylic acid vanillyl amide, benzyl nicotinate, β-butoxy ethyl ester nicotinate, capsaicin, zingerone, cantharis tincture, ichtammol, caffeine, tannic acid, α-borneol, tocopheryl nicotinate, inositol hexanicotinate, cyclandelate, cinnarizine, tolazoline, acetyl choline, verapamil, cepharanthin, and γ-oryzanol; skin astringents such as zinc oxide, and tannic acid; and anti-seborrheic agents such as sulfur and thianthol.

Examples of the vitamins include: vitamin A such as vitamin A oil, retinol, retinol acetate, and retinol palmitate; vitamin B including vitamin B2 such as riboflavin, riboflavin butyrate, and flavin adenine nucleotide, vitamin B6 such as pyridoxine hydrochloride, pyridoxine dioctanoate, and pyridoxine tripalmitate, vitamin B12 and its derivatives, and vitamin B15 and its derivatives; vitamin C such as L-ascorbic acid, L-ascorbic acid dipalmitic ester, sodium (L-ascorbic acid)-2-sulfate, and dipotassium L-ascorbic acid diphosphate; vitamin D such as ergocalciferol, and cholecarciferol; vitamin E such as α-tocopherol, β-tocopherol, γ-tocopherol, dl-α-tocopherol acetate, dl-α-tocopherol nicotinate, and dl-α-tocopherol succinate; vitamin H; vitamin P; nicotinic acids such as nicotinic acid, benzyl nicotinate, and nicotinic acid amide; pantothenic acids such as calcium pantothenate, D-pantothenyl alcohol, pantothenyl ethyl ether, and acetylpantothenyl ethyl ether; and biotin.

Examples of the amino acids include glycine, valine, leucine, isoleucine, serine, threonine, phenylaranine, alginine, lysine, aspartic acid, glutamic acid, cystine, cysteine, methionine, and tryptophan; and examples of the nucleic acids include deoxyribonucleic acid; and examples of the hormones include estradiol, and ethenyl estradiol.

Examples of the polymers for hair setting include amphoteric, anionic, cationic, and nonionic polymers, including: polymers of polyvinyl pyrrolidone type such as polyvinyl pyrrolidone, vinyl pyrrolidone/vinyl acetate copolymers; acidic polymers of vinyl ether type such as methyl vinyl ether/maleic acid anhydride alkyl half ester copolymer; polymers of acidic poly vinyl acetate type such as vinyl acetate/crotonic acid copolymer; acidic acrylic polymers such as (meth)acrylic acid/alkyl (meth)acrylate copolymer, (meth)acrylic acid/alkyl (meth)acrylate/alkyl acrylic amide copolymer; and amphoteric acrylic polymer such as N-methacryloylethyl-N,N-dimethylammonium α-N-methylcarboxybetaine/alkyl(meth)ahcrylate copolymer, hydroxypropyl (meth)acrylate/butylaminoethyl methacrylate/octyl amide of acrylic acid copolymer. It is also possible to preferably use naturally occurring polymers such as cellulose or derivatives thereof, keratin, collagen and derivatives thereof.

To be embraced in the meaning of "cosmetic preparation" in the present invention are: skin care products such as face lotion, milky lotion, cream, face cleansing cream, pack, oil liquid, massage materials, washing agent, deodorant, hand cream, and lip cream; makeup products such as makeup foundation, white powder, liquid foundation, oil-based foundation, rouge, eye shadow, mascara, eyeliner, eyebrow cosmetic, and lipstick; hairdressing products such as shampoo, rinse, treatment agent, setting agent; antiperspirant; and ultraviolet-ray protective cosmetic preparations such as sunscreen milky lotion, and sunscreen cream.
Further, it is possible to select a form of each of these cosmetic preparations, such as liquid, emulsion, cream, solid, paste, gel, powder, press, laminate, mousse, spray, stick, or pencil form, and the solid form or stick form is particularly preferable among them.

### EXAMPLES

Although the present invention will be explained hereinafter in more detail by describing Synthetic Examples, Comparative Synthetic Examples, Examples, and Comparative Examples, the present invention is not limited to these Examples and Comparative Examples. It is noted that "Me" means a methyl group, and "Bu" means a butyl group herein.

### (Synthetic Example 1)

Charged into a three-neck flask were 1.0 mole of glycerin monoallyl ether having the following structure, 2.0 mole of behenic acid, and 0.05 mass% of p-toluenesulfonic acid as a catalyst, followed by conduction of an esterification reaction at 190°C for 10 hours under nitrogen stream. Subsequently added thereinto was a sodium hydrogencarbonate water solution as a neutralizing agent in a manner to conduct a neutralization reaction at a temperature of 70°C for one hour, and to subsequently cool the product, which was then dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Glycerin monoallyl ether:

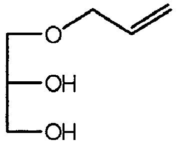

Methylhydrogen organopolysiloxane:

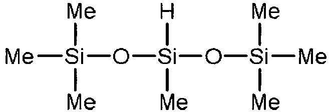

### (Synthetic Example 2)

Charged into a three-neck flask were 1.0 mole of the glycerin monoallyl ether, 2.0 mole of behenic acid, and 0.05 mass% of p-toluenesulfonic acid as a catalyst, followed by conduction of an esterification reaction at 190°C for 10 hours under nitrogen stream. Subsequently added thereinto was a sodium hydrogencarbonate water solution as a neutralizing agent in a manner to conduct a neutralization reaction at a temperature of 70°C for one hour, and to subsequently cool the product, which was then dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Methylhydrogen organopolysiloxane:

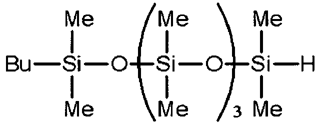

### (Synthetic Example 3)

Charged into a three-neck flask were 1.0 mole of the glycerin monoallyl ether, 2.0 mole of behenic acid, and 0.05 mass% of iron sulfate n-hydrate as a catalyst, followed by conduction of an esterification reaction at 190°C for 10 hours under nitrogen stream. Subsequently, the product was dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Methylhydrogen organopolysiloxane:

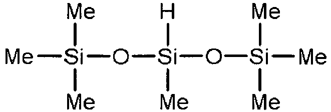

### (Synthetic Example 4)

Charged into a three-neck flask were 1.0 mole of the glycerin monoallyl ether, 2.0 mole of behenic acid, and 0.05 mass% of p-toluenesulfonic acid as a catalyst, followed by conduction of an esterification reaction at 190°C for 10 hours under nitrogen stream. Subsequently added thereinto was a sodium hydrogencarbonate water solution as a neutralizing agent in a manner to conduct a neutralization reaction at a temperature of 70°C for one hour, and to subsequently cool the product, which was then dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Methylhydrogen organopolysiloxane:

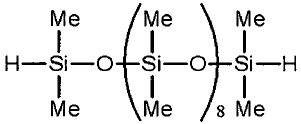

### (Synthetic Example 5)

Charged into a three-neck flask were 1.0 mole of triglycerin monoallyl ether having the following structure, 4.0 mole of behenic acid, and 0.05 mass% of p-toluenesulfonic acid as a catalyst, followed by conduction of an esterification reaction at 190°C for 10 hours under nitrogen stream. Subsequently added thereinto was a sodium hydrogencarbonate water solution as a neutralizing agent in a manner to conduct a neutralization reaction at a temperature of 70°C for one hour, and to subsequently cool the product, which was then dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Triglycerin monoallyl ether:

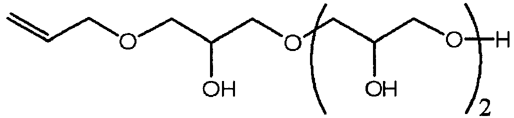

Methylhydrogen organopolysiloxane:

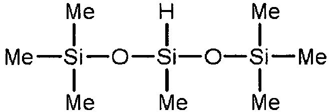

### (Synthetic Example 6)

Charged into a three-neck flask were 1.0 mole of triglycerin diallyl ether having the following structure, 3.0 mole of behenic acid, and 0.05 mass% of p-toluenesulfonic acid as a catalyst, followed by conduction of an esterification reaction at 190°C for 10 hours under nitrogen stream. Subsequently added thereinto was a sodium hydrogencarbonate water solution as a neutralizing agent in a manner to conduct a neutralization reaction at a temperature of 70°C for one hour, and to subsequently cool the product, which was then dissolved in tetrahydrofuran, followed by conduction of elimination of the solvent after filtration. To silicone-modify the thus obtained esterification reaction product, added thereinto were methylhydrogen organopolysiloxane having the following structure and 10% ethanol solution of chloroplatinic acid as a catalyst in an amount of 0.2 mass%, while using isopropanol as a solvent, followed by conduction of addition reaction at a temperature of 100°C for 8 hours under nitrogen stream, thereby obtaining a product.

Triglycerin diallyl ether:

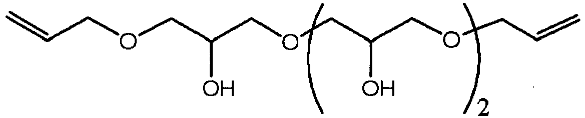

Methylhydrogen organopolysiloxane:

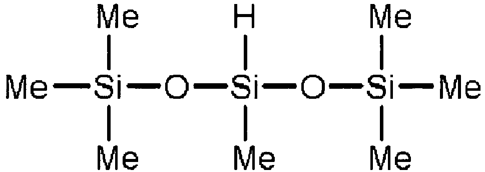

### (Comparative Synthetic Example 1)

Charged into a three-neck flask were 1.0 mole of the glycerin monoallyl ether, 2.0 mole of behenic acid, and 0.05 mass% of p-toluenesulfonic acid as a catalyst, followed by conduction of an esterification reaction at 190°C for 10 hours with stirring under nitrogen atmosphere. Subsequently added thereinto was a sodium hydrogencarbonate water solution as a neutralizing agent, followed by stirring at a temperature of 70°C for one hour, thereafter by dissolution of the product in tetrahydrofuran, and then by conduction of elimination of the solvent after filtration.

Compatibility of thickener with oil-based agent, and evaluation of composition:
To evaluate compatibilities of Synthetic Examples 1 to 6 and Comparative Example 1 with various oil-based agents, 1g of each thickener was added to 9g of each oil-based agent, and heated and dissolved together therewith at 80°C, followed by cooling at a room temperature, to finally evaluate the state of the applicable composition.

Evaluation of compatibility was determined as follows:
- Good:: dissolved
- Fair:: slightly turbid
- Poor:: insoluble

**[Table 1]**

| Appellation of used oil-based agent | Compatibility | | | | | | |
|---|---|---|---|---|---|---|---|
| | Synthetic Example | | | | | | Compara-tive Synthetic Example |
| | 1 | 2 | 3 | 4 | 5 | 6 | 1 |
| Decamethyl cyclopenta siloxane (KF-995) | Good | Good | Good | Good | Good | Good | Good |
| Dimethyl poly siloxane 2cs | Good | Good | Good | Good | Good | Good | Good |
| Dimethyl poly siloxane 6cs | Good | Good | Good | Good | Good | Good | Poor |
| Dimethyl poly siloxane 20cs | Fair | Good | Fair | Good | Fair | Good | Poor |
| Squalane | Good | Good | Good | Good | Good | Good | Good |
| Isononyl isononanoate | Good | Good | Good | Good | Good | Good | Good |
| Diisostearyl malate | Good | Good | Good | Good | Good | Good | Good |
| Glyceryl tri-2-ethyl hexanoate | Good | Good | Good | Good | Good | Good | Good |
| Jojoba oil | Good | Good | Good | Good | Good | Good | Good |
| Diglyceryl monoiso stearate | Good | Good | Good | Good | Good | Good | Good |
| Liquid paraffin | Good | Good | Good | Good | Good | Good | Good |

Used dimethylpolysiloxanes were KF-96-2cs, 6cs, and 20cs produced by Shin-Etsu Chemical Co., Ltd.

Evaluation of thickening effect was determined as follows:
- Excellent:: solidified
- Good:: thickened
- Fair:: partially separated
- Poor:: separated

**[Table 2]**

| Appellation of used oil-based agent | Thickening effect | | | | | | |
|---|---|---|---|---|---|---|---|
| | Synthetic Example | | | | | | Comparative Synthetic Example |
| | 1 | 2 | 3 | 4 | 5 | 6 | 1 |
| Decamethyl cyclopenta siloxane (KF-995) | Excellent | Excellent | Excellent | Excellent | Excellent | Good | Excellent |
| Dimethyl poly siloxane 2cs | Excellent | Excellent | Excellent | Excellent | Excellent | Good | Fair |
| Dimethyl poly siloxane 6cs | Excellent | Excellent | Excellent | Excellent | Excellent | Good | Poor |
| Dimethyl poly siloxane 20cs | Fair | Good | Fair | Good | Fair | Good | Poor |
| Squalane | Excellent | Excellent | Excellent | Excellent | Excellent | Good | Excellent |
| Isononyl isononanoate | Good | Good | Good | Good | Good | Good | Good |
| Diisostearyl malate | Excellent | Good | Excellent | Good | Excellent | Good | Excellent |
| Glyceryl tri-2-ethyl hexanoate | Excellent | Good | Excellent | Good | Excellent | Good | Excellent |
| Jojoba oil | Excellent | Good | Excellent | Good | Excellent | Good | Excellent |
| Diglyceryl monoiso stearate | Excellent | Excellent | Excellent | Excellent | Excellent | Good | Excellent |
| Liquid paraffin | Fair | Fair | Fair | Fair | Fair | Fair | Excellent |

As listed in Table 1 and Table 2, the thickeners of Synthetic Examples 1 to 6 were each high in compatibility with each nonvolatile silicone oil, in a manner to thicken or solidify it. Contrary, the thickener of Comparative Synthetic Example 1 was low in compatibility with each silicone oil, such that each oil-based agent was exuded, thereby failing to obtain a sufficient thickening effect.

The thickeners of Synthetic Examples 1 to 5 and Comparative Example 1 were used to prepare cosmetic preparations, respectively, followed by evaluations.

### (Example 1) Milky Lotion

| (Components) | | mass (%) |
|---|---|---|
| 1. | Silicone-modified wax obtained in Synthetic Example 1 | 2.0 |
| 2. | Dimethylpolysiloxane 2cs | 30.0 |
| 3. | Decamethylcyclopentasiloxane | 10.0 |
| 4. | Glyceryl trioctanoate | 5.0 |
| 5. | Polyethersilicone (note 1) | 5.0 |
| 6. | 1,3-butylene glycol | 5.0 |
| 7. | Antiseptic | q.s. |
| 8. | Perfume | q.s. |
| 9. | Purified water | 43.0 |

| | | |
|---|---|---|
| (note 1): KF-6017 (trade name) produced by Shin-Etsu Chemical Co., Ltd. | | |

### (Production Method)

A) Components 1 to 5 were heated and dissolved together into a solution.
B) Components 6, 7 and 9 were mixed, into which the solution obtained in A) was added and emulsified.
C) The emulsion obtained in B) was cooled, into which Component 8 was added to obtain a milky lotion.
The milky lotion as a product of the present invention obtained in the above manner was non-tacky and spread well. When applied to the skin, it gave a glossy finish.

### (Example 2) Water-in-Oil Type Cream

| (Components) | | mass (%) |
|---|---|---|
| 1. | Squalane | 10.0 |
| 2. | Decamethylcyclopentasiloxane | 7.0 |
| 3. | Dimethylpolysiloxane 2cs | 5.0 |
| | 4. Polyether-modified branched silicone (note 1) | 2.0 |
| 5. | Silicone-modified wax obtained in Synthetic Example 2 | 1.0 |
| 6. | Dipropylene glycol | 7.0 |
| 7. | Antiseptic | q.s. |
| 8. | Perfume | q.s. |
| 9. | Purified water | 68.0 |

| | | |
|---|---|---|
| (note 1): KF-6028 (trade name) produced by Shin-Etsu Chemical Co., Ltd. | | |

### (Production Method)

A) Components 1 to 5 were heatedly mixed with one another.
B) Components 6 to 9 were mixed with one another, and the mixture was added into the mixture obtained in A) and emulsified by stirring.
The water-in-oil type cream as a product of the present invention obtained in the above manner was non-tacky and spread well.

### (Example 3) Water-in-Oil Type Cream

| (Components) | | mass (%) |
|---|---|---|
| 1. | Silicone-modified obtained in Synthetic Example 3 | 2.0 |
| 2. | Dimethylpolysiloxane 6cs | 10.0 |
| 3. | Crosslinked type polyether-modified silicone (note 1) | 5.0 |
| 4. | Dipropylene glycol | 10.0 |
| 5. | Sodium citrate | 0.2 |
| 6. | Ethanol | 5.0 |
| 7. | Antiseptic | q.s. |
| 8. | Perfume | q.s. |
| 9. | Purified water | 67.8 |

| | | |
|---|---|---|
| (note 1): KSG-21 (trade name): produced by Shin-Etsu Chemical Co., Ltd. | | |

### (Production Method)

A) Components 1 to 3 were heated and dissolved together into a solution.
B) Components 4 to 9 were mixed with one another to prepare a solution, which was added into the solution obtained in A) and emulsified by stirring.
The water-in-oil type cream as a product of the present invention obtained in the above manner was non-greasy and non-tacky, and spread well. It gave finish having mat feel.

### (Example 4) Water-in-Oil Type Cream Makeup Base

| (Components) | | mass (%) |
|---|---|---|
| 1. | Crosslinked type polyether-modified silicone (note 1) | 4.0 |
| 2. | Crosslinked type dimethyloolysilicone (note 2) | 1.0 |
| 3. | Polyether-modified silicone (note 3) | 0.5 |
| 4. | Dimethylpolysiloxane(6 mm²/sec (25°C)) | 6.0 |
| 5. | Dimethylpolysiloxane(20 mm²/sec (25°C)) | 2.0 |
| 6. | Decamethylcyclopentasiloxane | 3.0 |
| 7. | Dispersion of titanium oxide in cyclopentasiloxane (note 4) | 10.0 |
| 8. | Silicone-modified wax obtained in Synthetic Example 2 | 1.0 |
| 9. | Dipropylene glycol | 5.0 |
| 10. | Sodium citrate | 0.2 |
| 11. | Methyl cellulose (2% aqueous solution) (note 5) | 2.5 |
| 12. | Ethanol | 3.0 |
| 13. | Antiseptic | q.s. |
| 14. | Perfume | q.s. |
| 15. | Purified water | 61.8 |

| | | |
|---|---|---|
| (note 1): KSG-21 (trade name): produced by Shin-Etsu Chemical Co., Ltd. (note 2): KSG-15 (trade name): produced by Shin-Etsu Chemical Co., Ltd. (note 3): KF-6017 (trade name): produced by Shin-Etsu Chemical Co., Ltd. (note 4): SPD-T1S (trade name): produced by Shin-Etsu Chemical Co., Ltd. (note 5): METOLOSE 65-SH4000 (trade name): produced by Shin-Etsu Chemical Co., Ltd. | | |

### (Production Method)

A) Components 1 to 8 were heatedly mixed with one another.
B) Components 9 to 15 were mixed with one another to prepare a solution, which was added into the solution obtained in A) and emulsified by stirring.
The water-in-oil type makeup base as a product of the present invention obtained in the above manner was non-greasy and non-tacky, and spread well. It gave moisturized and refreshing feel and mat finish.

### (Example 5) Oil-in-Water Type Cream

| (Components) | | mass (%) |
|---|---|---|
| 1. | Silicone-modified wax obtained in Synthetic Example 4 | 2.0 |
| 2. | Crosslinked type dimethylpolysilicone (note 1) | 15.0 |
| 3. | Decamethylcyclopentasiloxane | 10.0 |
| 4. | Dimethylpolysiloxane 20cs | 18.0 |
| 5. | Polyether-modified silicone (note 2) | 0.7 |
| 6. | Propylene glycol | 3.0 |
| 7. | Polyacrylamide mixture (note 3) | 0.8 |
| 8. | Xanthan gum(2% aqueous solution) | 8.0 |
| 9. | Antiseptic | q.s. |
| 10. | Perfume | q.s. |
| 11. | Purified water | 42.5 |

| | | |
|---|---|---|
| (note 1): KSG-16 (trade name) produced by Shin-Etsu Chemical Co., Ltd. (note 2): KF-6011 (trade name) produced by Shin-Etsu Chemical Co., Ltd. (note 3): SEPIGEL-305 (trade name) produced by SEPIC Co., Ltd.) | | |

### (Production Method)

A) Components 1 to 4 were heatedly mixed with one another.
B) Components 5 to 11 were mixed with one another to prepare a solution.
C) The mixture obtained in A) was added into the solution obtained in B) and emulsified by stirring.
It appeared that the oil-in-water type cream as a product of the present invention obtained in the above manner spread lightly on the skin and gave refreshing feel to the skin.

### (Example 6) Water-in-Oil Type Solid Cream

| (Components) | | mass (%) |
|---|---|---|
| 1. | Silicone-modified wax obtained in Synthetic | 30.0 |
| | Example 2 | |
| 2. | Dimethylpolysiloxane 20cs | 24.0 |
| 3. | Decamethylcyclopentasiloxane | 24.0 |
| 4. | Polyether-modified silicone (note 1) | 2.0 |
| 5. | 1,3-butylene glycol | 2.0 |
| 6. | Antiseptic | q.s. |
| 7. | Perfume | q.s. |
| 8. | Purified water | 18.0 |

| | | |
|---|---|---|
| (note 1): KF-6017 (trade name) produced by Shin-Etsu Chemical Co., Ltd. | | |

### (Production Method)

A) Components 1 to 4 were heated and dissolved together into a solution.
B) Components 5 to 8 were mixed with one another to prepare a solution, which was added into the solution obtained in A) and emulsified by stirring.
C) The emulsion of B) was filled into a container to prepare a product.
The water-in-oil type cream as a product of the present invention obtained in the above manner was non-greasy and non-tacky, and spread well on the skin, in spite of the large oil content.

### (Example 7) Lipstick

| (Components) | | mass (%) |
|---|---|---|
| 1. | Silicone-modified wax obtained in Synthetic Example 1 | 35.0 |
| 2. | Polyethylene wax | 5.0 |
| 3. | Squalane | 30.0 |
| 4. | Decamethylcyclopentasiloxane | 26.0 |
| 5. | Acrylsilicone resin (note 1) | 4.0 |
| 6. | Pigment | q.s. |
| 7. | Antiseptic | q.s. |
| 8. | Perfume | q.s. |

| | | |
|---|---|---|
| (note 1): KP-545 (trade name) produced by Shin-Etsu Chemical Co., Ltd. | | |

### (Production Method)

A) Components 1 to 4 were heated and dissolved together into a solution.
B) Components 5 to 7 were mixed with one another into a dispersion, which was added into the solution obtained in A), and stirred to make a homogeneous mixture.
C) Component 8 was added into the mixture obtained in B), and the obtained mixture was filled into a container to prepare a product.
The lipstick as a product of the present invention obtained in the above manner was non-greasy and non-tacky, and it spread lightly on the lips.

### (Example 8) Powder Foundation

| (Components) | | mass (%) |
|---|---|---|
| 1. | Sericite | 40.0 |
| 2. | Mica | 10.0 |
| 3. | Talc | balance |
| 4. | Titanium oxide | 10.0 |
| 5. | Titanium oxide fine powder | 5.0 |
| 6. | Magnesium stearate | 3.0 |
| 7. | Pigment | 4.2 |
| 8. | Silicone-modified wax obtained in Synthetic Example 3 | 1.0 |
| 9. | Squalane | 3.0 |
| 10. | Antiseptic | q.s. |
| 11. | Perfume | q.s. |

### (Production Method)

A) Components 8 to 11 were mixed with one another.
B) Components 1 to 7 were mixed with one another, into which the mixture obtained in A) was added and mixed homogeneously.
C) The mixture obtained in B) was press-molded by a mold, to obtain a powder foundation.
The powder foundation as a product of the present invention obtained in the above manner was non-tacky, spread well on the skin. Further, it appeared that the foundation gave a gloss finish.

### (Example 9) Cream Foundation

| (Components) | | mass (%) |
|---|---|---|
| 1. | Crosslinked type polyether-modified silicone (note 1) | 4.0 |
| 2. | Glyceryl trioctanoate | 3.0 |
| 3. | Diisostearyl malate | 5.0 |
| 4. | Decamethylcyclopentasiloxane | 6.0 |
| 5. | Silicone-modified wax obtained in Synthetic Example 5 | 2.0 |
| 6. | Fluorine-modified hybrid silicone composite powder (note 2) | 2.5 |
| 7. | Pigment | 8.0 |
| 8. | Acryl silicone resin (note 3) | 5.0 |
| 9. | Dipropylene glycol | 5.0 |
| 10. | Sodium citrate | 0.2 |
| 11. | Antiseptic | q.s. |
| 12. | Perfume | q.s. |
| 13. | Purified water | 59.3 |

| | | |
|---|---|---|
| (note 1): KSG-21 (trade name) produced by Shin-Etsu Chemical Co., Ltd. (note 2): KSP-200 (trade name) produced by Shin-Etsu Chemical Co., Ltd. (note 3): KP-545 (trade name) produced by Shin-Etsu Chemical Co., Ltd. | | |

### (Production Method)

A) Components 1 to 6 were heatedly mixed with one another.
B) Components 9 to 13 were mixed with one another to prepare a solution, which was added into the mixture obtained in A) and emulsified by stirring.
C) Components 7 and 8 were mixed with one another, which was added into the emulsion obtained in B) and made homogeneous.
It appeared that the cream foundation as a product of the present invention obtained in the above manner was non-tacky, spread well on the skin, and gave mat finish.

### (Example 10) Solid Foundation

| (Components) | | mass (%) |
|---|---|---|
| 1. | Silicone-modified wax obtained in Synthetic Example 1 | 30.0 |
| 2. | Polyethylene wax | 5.0 |
| 3. | Diisostearyl malate | 32.5 |
| 4. | Decamethylcyclopentasiloxane | 28.5 |
| 5. | Acrylic silicone resin (note 1) | 4.0 |
| 6. | Pigment | q.s. |
| 7. | Antiseptic | q.s. |
| 8. | Perfume | q.s. |

| | | |
|---|---|---|
| (note 1): KP-545 (trade name) produced by Shin-Etsu Chemical Co., Ltd. | | |

### (Production Method)

A) Components 1 to 4 were heated and dissolved together into a solution.
B) Components 5 to 7 were mixed with one another into a dispersion, which was added into the solution obtained in A), and stirred to make a homogeneous mixture.
C) Component 8 was added into the mixture obtained in B), and the obtained mixture was filled into a container to prepare a product.
It appeared that the foundation as a product of the present invention obtained in the above manner was non-greasy and non-tacky, and it spread well on the skin.

### (Example 11) Water-in-oil Type Compact Foundation

| (Components) | | mass (%) |
|---|---|---|
| 1. | Silicone-modified wax obtained in Synthetic Example 5 | 25.0 |
| 2. | Candellila wax | 5.0 |
| 3. | Glyceryl tri-2-ethylhexanoate | 24.0 |
| 4. | Decamethylcyclopentasiloxane | 22.0 |
| 5. | Acrylic silicone resin (note 1) | 4.0 |
| 6. | Trimethylsiloxy silicic acid (note 2) | 1.0 |
| 7. | Polyether-modified silicone (note 3) | 2.0 |
| 8. | Pigment | q.s. |
| 9. | 1,3-butylene glycol | 2.0 |
| 10. | Antiseptic | q.s. |
| 11. | Perfume | q.s. |
| 12. | Purified water | 15.0 |

| | | |
|---|---|---|
| (note 1): KP-545 (trade name) produced by Shin-Etsu Chemical Co., Ltd. (note 2): KF-7312J (trade name) produced by Shin-Etsu Chemical Co., Ltd. (note 3): KF-6017 (trade name) produced by Shin-Etsu Chemical Co., Ltd. | | |

### (Production Method)

A) Components 1 to 4, and 7 were heated and dissolved together into a solution.
B) Components 9, 10 and 12 were mixed with one another to prepare a solution, which was added into the solution obtained in A) and emulsified by stirring.
C) Components 5, 6 and 8 were mixed with one another into a dispersion, which was added into the emulsion obtained in B).
D) Component 11 was added into the mixture obtained in C), which was filled into a container to prepare a product.
The water-in-oil type compact foundation as a product of the present invention obtained in the above manner was non-greasy and non-sticky, and spread well on the skin, in spite of the large oil content.

### (Example 12) Eye Shadow

| (Components) | | mass (%) |
|---|---|---|
| 1. | Sericite | 40.0 |
| 2. | Mica | 10.0 |
| 3. | Talc | balance |
| 4. | Titanium oxide | 10.0 |
| 5. | Titanium oxide fine powder | 5.0 |
| 6. | Magnesium stearate | 3.0 |
| 7. | Pigment | q.s. |
| 8. | Octyldecanol | 3.0 |
| 9. | Glyceryl tri-2-ethylhexanoate | 8.0 |
| 10. | Silicone-modified wax obtained in Synthetic Example 3 | 2.0 |
| 11. | Antiseptic | q.s. |
| 12. | Perfume | q.s. |

### (Production Method)

A) Components 8 to 11 were heatedly mixed with one another.
B) Components 1 to 7 were mixed, into which the mixture obtained in A) was added and mixed homogeneously.
C) Component 12 was added into the mixture obtained in B).

It appeared that the eye shadow as a product of the present invention obtained in the above manner was non-greasy and spread well on the eyelids, and gave a glossy finish.

### (Example 13) Powder Eyebrow Cosmetic

| (Components) | | mass (%) |
|---|---|---|
| 1. | Silicone-modified wax obtained in Synthetic Example 2 | 3.0 |
| 2. | Jojoba oil | 5.0 |
| 3. | Glyceryl trioctanoate | 2.0 |
| 4. | Silicone-treated mica | 40.0 |
| 5. | Silicone-treated barium sulfate | 15.0 |
| 6. | Silicone-treated titanium oxide | 10.0 |
| 7. | Silicone-treated pigment | q.s. |
| 8. | Hybrid silicone composite powder (note 1) | 1.5 |
| 9. | Spherical polymethylsilsesquioxane powder (note 2) | 2.5 |
| 10. | Silicone-treated talc | balance |
| 11. | Antiseptic | q.s. |
| 12. | Perfume | q.s. |

| | | |
|---|---|---|
| (note 1): KSP-100 (trade name) produced by Shin-Etsu Chemical Co., Ltd. (note 2): KMP-590 (trade name) produced by Shin-Etsu Chemical Co., Ltd. | | |

### (Production Method)

A) Components 4 to 12 were homogeneously mixed with one another.
B) Components 1 to 3 were mixed with one another to prepare a solution, which was added into the mixture obtained in A) and made homogeneous.
C) The mixture obtained in B) was press molded in a mold, thereby obtaining a powder eyebrow cosmetic.
It appeared that the eyebrow cosmetic as a product of the present invention obtained in the above manner was non-tacky and spread well, and gave a glossy finish.

### (Example 14) Eyebrow Pencil

| (Components) | | mass (%) |
|---|---|---|
| 1. | Silicone-modified wax obtained in Synthetic Example 4 | 40.0 |
| 2. | Polyethylene wax | 10.0 |
| 3. | Jojoba oil | 40.0 |
| 4. | Decamethylcyclopentasiloxane | 5.0 |
| 5. | Acrylic silicone resin (note 1) | 5.0 |
| 6. | Pigment | q.s. |
| 7. | Antiseptic | q.s. |
| 8. | Perfume | q.s. |

| | | |
|---|---|---|
| (note 1): KP-545 (trade name) produced by Shin-Etsu Chemical Co., Ltd. | | |

### (Production Method)

A) Components 1 to 4 were heated and dissolved together into a solution.
B) Components 5 to 7 were mixed with one another into a dispersion, which was added into the solution obtained in A), and stirred to make a homogeneous mixture.
C) Component 8 was added into the mixture obtained in B), which was filled into a container to prepare a product.
The eyebrow pencil as a product of the present invention obtained in the above manner was not floury, was lubricious, and spread well.

### (Example 15) Hair Cream

| (Components) | | mass (%) |
|---|---|---|
| 1. | Silicone-modified wax obtained in Synthetic Example 4 | 2.0 |
| 2. | Diglyceryl monoisostearate | 5.0 |
| 3. | Decamethylcyclopentasiloxane | 8.0 |
| 4. | Stearyl trimethyl ammonium chloride | 1.5 |
| 5. | Glycerin | 3.0 |
| 6. | Propylene glycol | 5.0 |
| 7. | Hydroxyethylcellulose | 0.2 |
| 8. | Antiseptic | q.s. |
| 9. | Perfume | q.s. |
| 10. | Purified water | 75.3 |

### (Production Method)

A) Components 1 to 3 were heated and dissolved together into a solution.
B) Components 4 to 8 and 10 were homogeneously mixed with one another into a solution.
C) The solution obtained in B) was added into the solution obtained in A) and emulsified, followed by cooling and by subsequent addition of Component 9.
It appeared that the hair cream as a product of the present invention obtained in the above manner exhibited excellent effects in spread upon application onto hair, and in softness, smoothness, moisturized feel, and gloss of hair after the application, so that the hair cream was extremely excellent as a whole.

### (Example 16) Conditioning Mousse

| (Components) | | mass (%) |
|---|---|---|
| 1. | Silicone-modified wax obtained in Synthetic Example 2 | 0.5 |
| 2. | Diglyceryl monoisostearate | 2.0 |
| 3. | Crosslinked dimethylpolysiloxane (note 1) | 0.5 |
| 4. | Glyceryl trioctanoate | 1.5 |
| 5. | Glycerin | 3.0 |
| 6. | Stearyl dimehtyl benzyl ammonium chloride | 0.5 |
| 7. | Polyoxyethylene hydrogenated caster oil | 0.5 |
| 8. | Ethanol | 7.0 |
| 9. | Antiseptic | q.s. |
| 10. | Perfume | q.s. |
| 11. | Purified water | balance |
| 12. | Liquefied petroleum gas | 5.0 |

| | | |
|---|---|---|
| (note 1): KSG-16 (trade name) produced by Shin-Etsu Chemical Co., Ltd. | | |

### (Production Method)

A) Components 1 to 4 were heated and dissolved together into a solution.
B) Components 5 to 9 and 11 were homogeneously mixed with one another into a solution.
C) The solution obtained in B) was added into the solution obtained in A) and emulsified, followed by cooling and by subsequent addition of Component 10.
D) The product obtained in C) was filled into an aerosol can to obtain a conditioning mousse.
It appeared that the conditioning mousse as a product of the present invention obtained in the above manner was excellent in moisturized, soft and smooth feel to the hair, and it was non-greasy to give mat finish.

### (Example 17) Roll-On Type Antiperspirant

| (Components) | | mass (%) |
|---|---|---|
| 1. | Silicone-modified wax obtained in Synthetic Example 1 | 5.0 |
| 2. | Crosslinked polyether-modified silicone (note 1) | 20.0 |
| 3. | Liquid paraffin | 10.0 |
| 4. | Crosslinked dimethylpolysiloxane (note 2) | 15.0 |
| 5. | Decamethylcyclopentasiloxane | 30.0 |
| 6. | Aluminum zirconium tetrachlorohydrate | 20.0 |
| 7. | Perfume | q.s. |

| | | |
|---|---|---|
| (note 1): KSG-21 (trade name) produced by Shin-Etsu Chemical Co., Ltd. (note 2): KSG-15 (trade name) produced by Shin-Etsu Chemical Co., Ltd. | | |

### (Production Method)

A) Components 1 to 5 were heatedly mixed with one another.
B) Components 6 and 7 were added into the mixture obtained in A), and dispersed homogeneously.
The roll-on type antiperspirant obtained in the above manner spread well on the skin, was non-tacky and non-greasy, was free of degradation with temperature and time, and was extremely excellent in usability and stability.

### (Example 18) Water-in-Oil Type Antiperspirant

| (Components) | | mass (%) |
|---|---|---|
| 1. | Silicone-modified wax obtained in Synthetic Example 3 | 2.0 |
| 2. | Crosslinked polyether-modified silicone (note 1) | 7.0 |
| 3. | Decamethylcyclopentasiloxane | 7.0 |
| 4. | Liquid paraffin | 8.0 |
| 5. | 1,3-butylene glycol | 5.0 |
| 6. | Sodium citrate | 0.2 |
| 7. | Aluminum chlorohydrate | 20.0 |
| 8. | Perfume | q.s. |
| 9. | Purified water | 50.8 |

| | | |
|---|---|---|
| (note 1): KSG-21 (trade name) produced by Shin-Etsu Chemical Co., Ltd. | | |

### (Production Method)

A) Components 1 to 4 were heatedly mixed with one another.
B) Components 5, 6 and 8 were mixed, into which Components 7 and 8 were added and dissolved.
C) The mixture obtained in B) was added into the mixture obtained in A), and emulsified by stirring.
The water-in-oil type antiperspirant obtained in the above manner spread well on the skin, was non-tacky and non-greasy, was free of degradation with temperature and time, and was extremely excellent in usability and stability.

### (Example 19) Solid antiperspirant

| (Components) | | mass (%) |
|---|---|---|
| 1. | Silicone-modified wax obtained in Synthetic Example 5 | 22.0 |
| 2. | Polyethylene/polypropylene copolymer | 4.0 |
| 3. | Dimethylpolysiloxane (6 mm²/sec, (25°C)) | 22.0 |
| 4. | Decamethylcyclopentasiloxane | 22.0 |
| 5. | Crosslinked dimethylpolysiloxane (note 1) | 15.0 |
| 6. | Aluminum zirconium tetrachlorohydrate | 15.0 |
| 7. | Perfume | q.s. |

| | | |
|---|---|---|
| (note 1): KSG-16 (trade name) produced by Shin-Etsu Chemical Co., Ltd. | | |

### (Production Method)

A) Components 1 to 5 were mixed and heated into a homogeneous mixture.
B) Components 6 and 7 were added into the mixture obtained in A) and dispersed.
C) The dispersion obtained in B) was filled into a container to prepare a product.
The solid antiperspirant obtained in the above manner spread well on the skin, was non-tacky and non-greasy, was free of degradation with temperature and time, and was extremely excellent in usability and stability.

### (Example 20) Solid Antiperspirant

| (Components) | | mass (%) |
|---|---|---|
| 1. | Silicone-modified wax obtained in Synthetic Example 4 | 25.0 |
| 2. | Polyethylene wax | 8.0 |
| 3. | Dimethylpolysiloxane(6 mm²/sec (25°C)) | 25.0 |
| 4. | Decamethylcyclopentasiloxane | 22.0 |
| 5. | Crosslinked dimethylpolysiloxane (note 1) | 5.0 |
| 6. | Aluminum zirconium tetrachlorohydrate(glycine salt) | 15.0 |
| 7. | Perfume | q.s. |

| | | |
|---|---|---|
| (note 1): KSG-16 (trade name) produced by Shin-Etsu Chemical Co., Ltd. | | |

### (Production Method)

A) Components 1 to 5 were mixed and heated into a homogeneous mixture.
B) Components 6 and 7 were added into the mixture obtained in A) and dispersed.
C) The dispersion obtained in B) was filled into a container to prepare a product.
The solid antiperspirant obtained in the above manner spread well on the skin, was non-tacky and non-greasy, was free of degradation with temperature and time, and was extremely excellent in usability and stability.

### (Example 21) UV Cut Water-in-Oil Type Cream

| (Components) | | mass (%) |
|---|---|---|
| 1. | Silicone-treated zinc oxide | 20.0 |
| 2. | Acryl-modified silicone resin (note 1) | 12.0 |
| 3. | Decamethylcyclopentasiloxane | 20.0 |
| 4. | Glyceryl trioctanoate | 3.0 |
| 5. | Silicone-modified wax obtained in Synthetic Example 2 | 2.0 |
| 6. | Crosslinked polyether-modified silicone (note 2) | 5.0 |
| 7. | Polyether-modified silicone (note 3) | 1.0 |
| 8. | Alkyl- and polyether-modified silicone (note 4) | 1.0 |
| 9. | Methoxy octylcinnamate | 6.0 |
| 10. | Sodium citrate | 0.2 |
| 11. | Dipropylene glycol | 3.0 |
| 12. | Antiseptic | q.s. |
| 13. | Perfume | q.s. |
| 14. | Purified water | 26.8 |

| | | |
|---|---|---|
| (note 1): KP-545 (trade name) produced by Shin-Etsu Chemical Co., Ltd. (note 2): KSG-21 (trade name) produced by Shin-Etsu Chemical Co., Ltd. (note 3): KF-6017 (trade name) produced by Shin-Etsu Chemical Co., Ltd. (note 4): KF-6026 (trade name) produced by Shin-Etsu Chemical Co., Ltd. | | |

### (Production Method)

A) Part of component 3 and components 4 to 9 were heatedly mixed with one another.
B) Components 10 to 12, and 14 were mixed with one another, and the mixture was added into the mixture obtained in A) and emulsified by stirring.
C) Components 1 and 2 and the remainder of component 3 were mixed with one another into a dispersion, which was added together with component 13 into the emulsion obtained in B), to make a homogeneous mixture.
The water-in-oil type of UV cut cream obtained in the above manner spread well on the skin, was non-tacky and non-greasy, exhibited transparent feel, was free of degradation with temperature and time, and was extremely excellent in usability and stability.

### (Example 22) UV Cut Water-in-Oil Type Milky Lotion

| (Components) | | mass (%) |
|---|---|---|
| 1. | Dimethylpolysiloxane(6 mm²/sec (25°C)) | 5.0 |
| 2. | Crosslinked polyether-modified silicone (note 1) | 5.0 |
| 3. | Glyceryl trioctanoate | 2.0 |
| 4. | Silicone-modified wax obtained in Synthetic Example 3 | 1.0 |
| 5. | Polyether-modified silicone (note 2) | 1.0 |
| 6. | Dispersion of titanium oxide in | 30.0 |
| | decamethylcyclopentasiloxane (note 3) | |
| 7. | Dispersion of zinc oxide in decamethylcyclopentasiloxane (note 4) | 30.0 |
| 8. | Dipropylene glycol | 3.0 |
| 9. | Sodium citrate | 0.2 |
| 10. | Antiseptic | q.s. |
| 11. | Perfume | q.s. |
| 12. | Purified water | 22.8 |

| | | |
|---|---|---|
| (note 1): KSG-21 (trade name) produced by Shin-Etsu Chemical Co., Ltd. (note 2): KF-6017 (trade name) produced by Shin-Etsu Chemical Co., Ltd. (note 3): SPD-T1S (trade name) produced by Shin-Etsu Chemical Co., Ltd. (note 4): SPD-Z1 (trade name) produced by Shin-Etsu Chemical Co., Ltd. | | |

### (Production Method)

A) Components 1 to 5 were heatedly mixed with one another.
B) Components 8 to 10 and 12 were mixed with one another to prepare a solution, which was added into the mixture obtained in A), and emulsified by stirring.
C) Components 6, 7 and 11 were added into the emulsion obtained in B) and made homogeneous.
The water-in-oil type of UV cut milky lotion obtained in the above manner spread well on the skin, was non-tacky and non-greasy, exhibited transparent feel together with excellent cosmetic retention, was free of degradation with temperature and time, and was extremely excellent in usability and stability.

### (Example 23) UV Cut Oil-in-Water Type Cream

| (Components) | | mass (%) |
|---|---|---|
| 1. | Crosslinked organopolysiloxane (note 1) | 5.0 |
| 2. | Cetyl isooctanoate | 5.0 |
| 3. | Silicone-modified wax obtained in Synthetic Example 5 | 1.0 |
| 4. | Dispersion of titanium oxide in decamethylcyclopentasiloxane (note 2) | 15.0 |
| 5. | Polyether-modified silicone (note 3) | 1.0 |
| 6. | Polyether-modified silicone (note 4) | 1.0 |
| 7. | Acrylic acid amide mixture (note 5) | 2.0 |
| 8. | Propylene glycol | 5.0 |
| 9. | Methylcellulose (2% aqueous solution) (note 6) | 5.0 |
| 10. | Antiseptic | q.s. |
| 11. | Perfume | q.s. |
| 12. | Purified water | 60.0 |

| | | |
|---|---|---|
| (note 1): KSG-18 (trade name) produced by Shin-Etsu Chemical Co., Ltd. (note 2): SPD-T1S (trade name) produced by Shin-Etsu Chemical Co., Ltd. (note 3): KF-6027 (trade name) produced by Shin-Etsu Chemical Co., Ltd. (note 4): KF-6011 (trade name) produced by Shin-Etsu Chemical Co., Ltd. (note 5): SEPIGEL 305 (trade name) produced by SEPIC Co., Ltd. (note 6): METOLOSE SM-4000 (trade name) produced by Shin-Etsu Chemical Co., Ltd. | | |

### (Production Method)

A) Components 5 to 8, 10 and 12 were mixed with one another.
B) Components 1 to 3 were heatedly mixed with one another into a mixture, which was added into the mixture obtained in A, and emulsified by stirring.
C) Component 4 was added into the emulsion obtained in B), into which Components 9 and 10 were added and made homogeneous.
The oil-in-water type of UV cut cream obtained in the above manner spread well on the skin, was non-tacky and non-greasy, exhibited transparent feel together with excellent cosmetic retention, was free of degradation with temperature and time, and was extremely excellent in usability and stability.

### (Comparative Example 1) Solid Foundation

Prepared was a solid foundation in the same manner as Example 10 except that the thickener obtained in Comparative Synthetic Example 1 was used instead of the component 1: silicone-modified wax in Example 10. The thus obtained solid foundation was tackier and worse in spread than the foundation in Example 10.

It is noted that the present invention is not limited to the above embodiments. The embodiments are illustrative, and whatever have substantially the same configuration as the technical concept recited in the claims of the present application and exhibit the same functions and effects, are embraced within the technical scope of the present invention.

## Claims

1. A silicone-modified wax obtained by subjecting an esterification reaction product obtained from a polyhydric alcohol having one or two alkenyl groups in one molecule and a higher fatty acid, and an organohydrogenpolysiloxane containing at least one methyl group, to a hydrosilylation reaction;
wherein the silicone-modified wax is solid or in a grease state exhibiting a thixotropy, at 25°C.

2. The silicone-modified wax according to claim 1, wherein the polyhydric alcohol having one or two alkenyl groups in one molecule is a compound having a linear or branched polyglycerin structure;
wherein the higher fatty acid is a saturated linear higher fatty acid having 12 to 30 carbon atoms; and
wherein the organohydrogenpolysiloxane is represented by the following general formula (A), 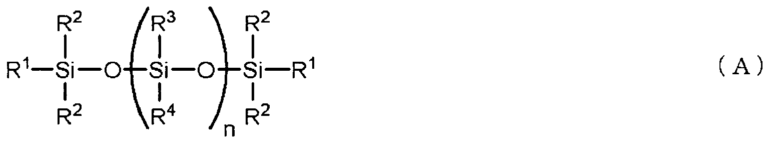 wherein R¹, R², R³, and R⁴ each independently represent:
a hydrogen atom; or
an identical or different linear or branched alkyl group or alkoxy group having 1 to 22 carbon atoms, or an identical or different aryl group having 6 to 22 carbon atoms, or an identical or different fluorine-substituted alkyl group, such that each group may have a coupled group or substitutional group having a branched polyglycerol chain;
wherein at least one of R¹ and R³ represents a hydrogen atom, at least one of R² and R⁴ represents a methyl group, and n represents an integer of 0 to 100.

3. The silicone-modified wax according to claim 1 or 2, wherein the silicone-modified wax has a melting point between 40°C and 100°C.

4. A composition containing the silicone-modified wax according to any one of claims 1 to 3 in an amount between 5 and 40 mass%, wherein the composition is in a paste state or solid state at 25°C and at an ordinary pressure.

5. A cosmetic preparation containing the silicone-modified wax according to any one of claims 1 to 3.

6. A production method of a silicone-modified wax, comprising:
(I) a step of subjecting a polyhydric alcohol having one or two alkenyl groups in one molecule and a higher fatty acid, to an esterification reaction in the presence of a catalyst, thereby obtaining an esterification reaction product; and
(II) a step of subjecting the esterification reaction product and an organohydrogenpolysiloxane containing at least one methyl group, to a hydrosilylation reaction in the presence of a catalyst.

7. The production method of a silicone-modified wax according to claim 6, further comprising the steps of:
adopting, as the polyhydric alcohol having one or two alkenyl groups in one molecule, a compound having a linear or branched polyglycerin structure,;
adopting, as the higher fatty acid, a saturated linear higher fatty acid having 12 to 30 carbon atoms; and
adopting, as the organohydrogenpolysiloxane, a compound represented by the following general formula (A), 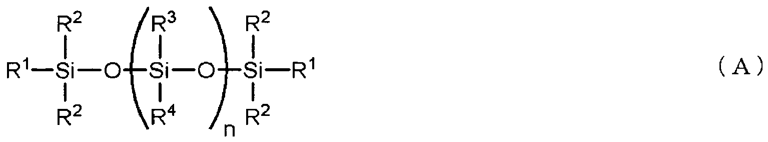
wherein R¹, R², R³, and R⁴ each independently represent:
a hydrogen atom; or
an identical or different linear or branched alkyl group or alkoxy group having 1 to 22 carbon atoms, or an identical or different aryl group having 6 to 22 carbon atoms, or an identical or different fluorine-substituted alkyl group, such that each group may have a coupled group or substitutional group having a branched polyglycerol chain;
wherein at least one of R¹ and R³ represents a hydrogen atom, at least one of R² and R⁴ represents a methyl group, and n represents an integer of 0 to 100.
